# EUROPEAN PATENT APPLICATION

(11) **EP 2 374 479 A2**
(43) Date of publication of application: **12.10.2011**
(21) Application number: 09833598.7
(22) Date of filing: 10.12.2009
(51) Int. Cl.: A61K 47/48, A61K 31/519, A61K 9/22

(54) **CONTROLLED-RELEASE COMPOSITION FOR PRODUCING SUSTAINED-RELEASE PREPARATION CONTAINING UDENAFIL**

(30) Priority: 17.12.2008 KR 20080128499
(71) Applicant: Dong-A Pharm.Co., Ltd., Seoul 130-070 (KR)
(72) Inventor: YOO, Moo-Hi, Seoul 135-807 (KR); CHA, Bong-Jin, Anyang-si Gyeonggi-do 431-080 (KR); KIM, Jeong-Hoon, Seongnam-si Gyeonggi-do 463-727 (KR); JANG, Sun-Woo, Seoul 140-040 (KR); HAN, Sang-Dug, Yongin-si Gyeonggi-do 446-905 (KR)
(74) Representative: Albrecht, Thomas
(86) International application number: PCT/KR2009/007382
(87) International publication number: WO 2010/071320

(57) **Abstract**

This invention relates to a controlled-release composition for producing a sustained-release preparation containing udenafil, including (A) udenafil and a pharmaceutically acceptable salt, (B) a solubility modulator, (C) an adsorbent, and (D) a hydrophilic polymer. This controlled-release composition for producing a sustained-release preparation containing udenafil releases drugs constantly regardless of the pH level in the gastrointestinal tract, and thus freely controls the drug release time within the range of 3∼24 hours, and reduces the variability in the effect of drugs among individuals. Also, this composition can be produced into a sustained-release preparation which has an optimum condition for expressing the effect of drugs in the treatment of diseases including pulmonary arterial hypertension, hepatic portal vein hypertension, benign prostatic hyperplasia, and the like, which can be treated by udenafil and which requires the long-term drug administration. Also, this composition can control the release of drugs in accordance with the time taken for the absorption thereof when the drugs are applied to a living body, and thus can be valuably used in preventing and treating erectile dysfunction.

## Description

### Technical Field

The present invention relates to a controlled-release composition for producing a sustained-release preparation containing udenafil.

### Background Art

As represented by Formula 1 below, a pyrazolopyrimidinone compound (3-(1-methyl-7-oxo-3-propyl-4,7-dihydro-1H-pyrazolo[4,3-d]pyrimidin-5-yl)-N-[2-(1-methylpyrrolidin-2-yl)ethyl]-4-propoxybenzenesulfonamide, hereinafter referred to as 'udenafil'), which is a kind of phosphodiestrase type-5 inhibitor (hereinafter referred to as 'PDE-5 inhibitor'), is commercially available as a therapeutic agent for erectile dysfunction.

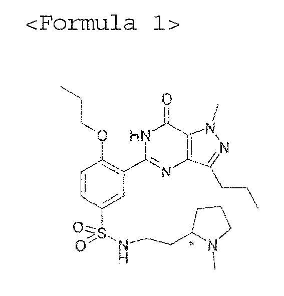

Udenafil has high selectivity for Pre-5 with exhibiting strong inhibitory activity therefor, is rapidly absorbed, and has high bioavailability and a large volume of distribution in vivo, and the half life thereof is about three times longer than that of sildenafil or vardenafil which is a drug of the same mechanism.

PDE-5 inhibitors cause side effects such as facial flushing, headache, eye redness, non-redness, etc., in terms of their pharmacological mechanisms. Among these, udenafil did not generate serious abnormal reactions by drugs in phase 1 clinical tests (Koreans and Caucasians), and caused only mild illness, if any. Also in the type of side effects observed in phase 2 and 3 clinical tests the extent and frequency of side effects were lower than when using conventional oral therapeutic agents for erectile dysfunction and the drug discontinuation rate was the lowest during the clinical tests. Hence, udenafil is considered to be a safe drug.

Korean Patent Nos. 792126 and 496372 disclose novel medical uses of PDE-5 inhibitor based drugs for treating pulmonary arterial hypertension, hepatic portal vein hypertension, and benign prostatic hyperplasia.

However, because most of the above diseases are chronic, therapeutic agents applied thereto should be inevitably administered for a long period of time in terms of their pharmacological mechanisms.

Udenafil, which is safer than other PDE-5 inhibitor based drugs, does not need special dosage forms in order for it to be used as a therapeutic agent for erectile dysfunction that is administered as necessary. However, upon long-term administration for treating the above indications which require daily administration, there may be a concern about causing side effects. Accordingly, when udenafil is developed in the form of a preparation adapted for the above indications, it is important that udenafil be formulated into a preparation such that it is controllably released from the drug dosage form thus reducing initial burst and also is continuously released for a period of time during which it may be absorbed in vivo, thereby sufficiently exhibiting the effects of the drug even when taken once a day and minimizing generable side effects.

Furthermore, people associated with the above diseases are mainly the elderly, and patients who take an antacid or the like have high pH in the stomach and patients who suffer from gastric ulcers or Zollinger-Ellison syndrome have low pH in the stomach. In cases where such patients are afflicted with pulmonary arterial hypertension, hepatic portal vein hypertension or benign prostatic hyperplasia, the sustained-release preparation containing udenafil is required to constantly release the drug regardless of the pH level in the gastrointestinal tract so that the release time of drug is freely adjusted in the range of 3∼24 hours.

Conventionally, controlled-release preparations of udenafil are not disclosed. Only in the case of sildenafil which is a drug of the same mechanism, some controlled-release preparations are proposed.

Korean Patent Laid-open Publication No. 2004-83492 discloses an osmotic delivery system, and Korean Patent Laid-open Publication No. 2002-70330 discloses a hydrogel-based drug dosage form. International Publication No. 2007/057762 discloses a controlled-release dosage form, in which the controlled-release preparation is formulated using complicated production processes including several tablet compressions, insoluble water-permeable coating and final laser perforation. Also, Korean Patent Laid-open Publication Nos. 2001-36527 and 2007-100023 disclose only a fast-dissolving dosage form which releases almost all of sildenafil within several minutes.

Consequently, the present inventors have studied the production of sustained-release preparations containing udenafil and have found the fact that the composition containing udenafil according to the present invention may exhibit controlled-release effects and may freely control the release of drug without being affected by any pH in vivo, and also the controlled-release composition containing udenafil may be easily formulated into a sustained-release preparation of udenafil which is able to reduce the probability of generating side effects even upon long-term administration to treat pulmonary arterial hypertension, hepatic portal vein hypertension and benign prostatic hyperplasia and to prevent and treat erectile dysfunction, thereby completing the controlled-release composition containing udenafil.

### Disclosure

### Technical Problem

Accordingly, an object of the present invention is to provide a controlled-release composition for producing a sustained-release preparation containing udenafil, which controls the release of udenafil in vivo regardless of the pH level in the gastrointestinal tract and thus may sustain its effect in order to treat pulmonary arterial hypertension, hepatic portal vein hypertension and benign prostatic hyperplasia and to prevent and treat erectile dysfunction.

### Technical Solution

The present invention pertains to a controlled-release composition for producing a sustained-release preparation containing udenafil.

Udenafil may be efficiently used to treat pulmonary arterial hypertension, hepatic portal vein hypertension, and benign prostatic hyperplasia and to prevent and treat erectile dysfunction.

However, because the preparations for use in treating the above indications may cause side effects due to long-term administration, the release of udenafil in vivo should extend and also extension thereof should be adjusted in order to develop udenafil in a preparation form adapted for the above indications. In addition, people associated with the above diseases are mainly the elderly, and patients who take an antacid or the like have high pH in the stomach, and patients who suffer from gastric ulcers or Zollinger-Ellison syndrome have low pH in the stomach. Upon treating the diseases of such patients, the sustained-release preparation containing udenafil should be able to constantly release the drug regardless of the pH level in the gastrointestinal tract. Even when using typical production methods, controlled-release type sustained-release preparations should be able to be produced.

Therefore, the controlled-release composition for producing a sustained-release preparation containing udenafil according to the present invention should be able to 1) uniformly release a drug regardless of the pH level in the gastrointestinal tract, and 2) to design the production of a controlled-release type sustained-release preparation using a typical production method.

In order to accomplish the above objects, the present invention provides a controlled-release composition for producing a sustained-release preparation containing udenafil, which comprises (A) udenafil and its pharmaceutically acceptable salt, (B) a solubility modulator, (C) an adsorbent, and (D) a hydrophilic polymer.

Hereinafter, a detailed description will be given of the present invention.

In the formation of a controlled-release composition for producing a sustained-release preparation containing udenafil according to the present invention, the controlled-release composition containing udenafil includes a solubility modulator. The solubility modulator according to the present invention may include an organic acid, such as citric acid, malic acid, adipic acid, maleic acid, ascorbic acid, succinic acid, and tartaric acid. Particularly useful is citric acid.

Most PDE-5 inhibitors are weakly basic drugs and have high solubility under acidic conditions and the solubility thereof decreases in proportion to an increase in pH. Also udenafil which is a weakly basic drug has high solubility such that almost all of the administered amount is dissolved under acidic conditions, and may be formulated into a preparation without any limitation upon production of an immediate-release preparation. However, in cases where the PDE-5 inhibitors are produced in the form of a controlled-release type sustained-release preparation, a dissolution rate on the release control membrane may vary depending on a difference in solubility at different pH values.

Thus in the present invention, the solubility modulator is added to a hydrated gel matrix so that the drug is continuously released in the gastrointestinal tract regardless of the pH level, thus solubilizing the hydrated gel matrix to result in high-concentration udenafil.

Use of an organic acid as a solubilizer for a weakly basic drug such as udenafil which is an active component of the present invention is disclosed in International Publication Nos. 2001/47500 and 97/18814. However, when granules are formed by mixing udenafil which is weakly basic with an organic acid according to the above known techniques, they are very sticky and thus undesirably make it impossible to form granules and tablets thereof.

Hence, with the goal of solving the above problems in the present invention, an adsorbent, in lieu of the binder, is contained in the controlled-release composition for producing a sustainted-release preparation containing udenafil.

To solve the stickiness problem to some degree using a typical excipient, the amount of excipient should be increased. In this case, the size of dosage form is remarkably increased to the extent that patients have difficulty in taking it.

Thus in the present invention, in order to keep the size of tablets small and to solve the stickiness problems of granules, the adsorbent such as silicon dioxide, calcium silicate, talc, and aluminum magnesium metasilicate is used, and the stickiness of granules themselves functions as a binder, and thus good shape of granules may be maintained after drying, even without the use of the binder.

In order to control the release of udenafil, according to the present invention, a high-viscosity hydrophilic polymer is contained in the controlled-release composition for producing a sustained-release preparation containing udenafil. The high-viscosity hydrophilic polymer according to the present invention includes hydroxyethylcellulose, hydroxypropylcellulose, hydroxypropylmethylcellulose, polyethyleneoxide, xanthan gum, guar gum, locust bean gum, sodium alginate, etc. Particularly useful is hydroxypropylmethylcellulose or polyethyleneoxide.

In the present invention, the high-viscosity hydrophilic polymer forms a gel upon contact between udenafil that is an active component and water, and thus acts as a barrier for blocking the discharge of the active component from the dosage form. The dissolution time of the pharmaceutical composition according to the present invention may be freely adjusted in the range of 3∼24 hours depending on the kind of polymer, the weight ratio and the viscosity.

According to the present invention, the controlled-release composition for producing a sustained-release preparation containing udenafil is uniformly dissolved in the gastrointestinal tract regardless of the pH level so that it is uniformly absorbed by the entire region of the gastrointestinal tract. Even in the case of patients having pH dependency, including the elderly or patients who take an antacid or the like or suffer from gastric ulcers or Zollinger-Ellison syndrome, the composition according to the present invention can be uniformly dissolved regardless of the pH level in the gastrointestinal tract, so that it is uniformly absorbed by the entire region of the gastrointestinal tract, thus reducing the variability among individuals and preventing initial burst upon release of drug. Thereby the excessive maximum concentration (Cmax) in the blood may be decreased, thus reducing drug-related side effects.

The composition according to the present invention may further include a pore forming agent. The pore forming agent used in the present invention includes saccharides such as lactose, sucrose, mannitol, erythritol, etc., water-soluble salts such as sodium chloride, potassium chloride, etc., or polymers such as polyethyleneoxide, etc. Particularly useful is polyethyleneoxide. The pore forming agent enables the formation of pores for water movement when the high-viscosity hydrophilic polymer forms a gel, thus further controlling the release of the drug.

Also the composition according to the present invention may further include a swelling agent. The swelling agent used in the present invention includes croscarmellose sodium, sodium starch glycolate, etc., and sodium starch glycolate may be particularly used. When the swelling agent comes into contact with water, it rapidly absorbs water so that the water-soluble polymer is rapidly gelled up to the inside thereof and simultaneously tablets are expanded, thus aiding the continuous and uniform release of drug from the dosage form.

According to the present invention, the controlled-release composition may further include a diluent or a lubricant. The dilutent may include lactose, mannitol, microcrystalline cellulose and mixtures thereof, and Cellactose is a representative commercially available product. The diluent enables the formation of a preparation having a predetermined size and imparts sufficient bondability upon tabletting. Also, the lubricant may include magnesium stearate, calcium stearate, stearic acid, talc, aerosol, castor oil, and sodium stearyl fumarate, and imparts sufficient fluidity upon tabletting and prevents bonding between the punch and the die.

In addition, the present invention provides a method of producing the controlled-release composition for producing a sustained-release preparation containing udenafil.

The method of producing the controlled-release composition for producing a sustained-release preparation containing udenafil according to the present invention includes adsorbing a mixture solution comprising udenafil or its salt and the solubility modulator on the surface of the absorbent, thus forming adsorbed granules, and mixing the adsorbed granules with the water-soluble polymer thus producing the controlled-release preparation.

In the method of producing the controlled-release pharmaceutical composition for a sustained-release preparation containing udenafil according to the present invention, when the adsorbed granules and the polymer are mixed to produce the controlled-release preparation, a pore forming agent, a swelling agent, a diluent or a lubricant may be further added.

### Advantageous Effects

According to the present invention, a controlled-release composition for producing a sustained-release preparation containing udenafil enables a drug to be constantly released regardless of the pH level, and can be uniformly absorbed in the entire region of the gastrointestinal tract and the release time of the drug can be freely adjusted in the range of 3∼24 hours. In particular, the variability among individuals can be reduced, and initial burst can be prevented upon release of the drug thus decreasing the excessive Cmax in the blood and reducing drug-related side effects.

Also the controlled-release composition for producing a sustained-release preparation containing udenafil according to the present invention can solve the stickiness problem of granules caused by a main component and an organic acid during the production process and can thus reduce the dosage form of tablets, and can be easily produced.

### Description of Drawings

FIG. 1 shows the results of dissolution test of the controlled-release preparations of Examples 1 to 4;
FIG. 2 shows the results of dissolution test of the preparations of Example 4 and Comparative Examples 3 to 6; and
FIG. 3 shows the results of dissolution test of the controlled-release preparation of Example 4 at various pH values.

### Best Mode

Hereinafter, the present invention is described in detail via the examples.

The following examples are merely set forth to illustrate the present invention but are not construed to limit the present invention.

### <Examples 1∼4> Production of controlled-release composition for producing sustained-release preparation containing udenafil and production of preparation therefrom according to the present invention

### 1) Production of adsorbed granules for controlled-release composition

Adsorbed granules for producing a udenafil controlled-release composition were prepared from the components of Examples 1 to 4 shown in Table 1 below.

Specifically, udenafil and citric acid were dissolved in an appropriate amount of water, after which silicon dioxide was introduced into a high-rate mixer and the above udenafil-citric acid solution was slowly added in droplets thereto, so that the above components were uniformly adsorbed onto the surface of silicon dioxide. The adsorbed granules were placed in a high-rate dryer and dried at 60°C for 30 min and then milled.

**TABLE 1**

| Unit : g | | | | |
|---|---|---|---|---|
| unit: g | Ex.1 | Ex.2 | Ex.3 | Ex.4 |
| Udenafil | 75.0 | | | |
| Anhydrous Citric Acid | 9.4 | 11.3 | 22.5 | 75.0 |
| Silicon Dioxide | 112.5 | | | |

### 2) Production of controlled-release composition using adsorbed granules

The adsorbed granules obtained in 1) were mixed with components of Examples 1 to 4 shown in Table 2 below including hydroxypropylmethylcellulose as a water-soluble polymer and Cellactose80 as a diluent by means of a mixer, after which sodium stearyl fumarate was further added and mixed for 5 min, thus obtaining a controlled-release composition for producing a sustained-release preparation containing udenafil.

**TABLE 2**

| unit: g | Ex.1 | Ex.2 | Ex.3 | Ex.4 |
|---|---|---|---|---|
| Adsorbed Granules | 196.9 | 198.8 | 210.0 | 262.5 |
| HPMC 4000SR (15%) | 41.6 | 42.0 | 44.4 | 55.5 |
| Cellactose80 | 28.1 | 28.4 | 30.0 | 112.5 |
| Sodium Stearyl Fumarate | 2.7 | 2.7 | 2.9 | 3.6 |

### 3) Production of sustained-release preparation using controlled-release composition

The controlled-release composition obtained in 2) for producing a sustained-release preparation was molded into 10KP using a tablet compressor so that the amount of main component was 75 mg per tablet, thus producing the sustained-release preparation.

### <Examples 5∼12> Production of controlled-release composition for producing sustained-release preparation containing udenafil using various polymers and production of preparation therefrom according to the present invention

The adsorbed granules were formed in the same manner as in 1) of Examples 1 to 4, after which udenafil controlled-release preparations were produced in the same manner as in 2) and 3) of Examples 1 to 4 using components shown in Table 3 below and defined as Examples 5 to 12.

**TABLE 3**

| unit : g | Ex. 5 | Ex. 6 | Ex.7 | Ex. 8 | Ex. 9 | Ex.10 | Ex. 11 | Ex.12 |
|---|---|---|---|---|---|---|---|---|
| Udenafil | 75 | | | | | | 100 | |
| Anhydrous Citric Acid | 75 | | | | | | 100 | |
| Silicon Dioxide | 112.5 | | | | | | 150 | |

| Polymer | 7.5% HPMC 4000SR 27 | 40% PEO WSR303 205 | 30% PEO 15% WSR303 154 | 15 % PEO WSR303 48 | 10% PEO WSR303 31 | 10% PEO WSR301 31 | 10% PEO WSR 41.3 | 10%PEO WSR 301 44.0 |
|---|---|---|---|---|---|---|---|---|
| PEG6000 | - | 41 | 30 | 9 | 6.2 | 6.2 | | |
| Primojel | - | - | - | - | - | - | - | 44.0 |
| Cellactose80 | 66 | - | - | - | - | - | - | - |
| Sodium Stearyl Fumarate | 3.6 | 5 | 4.5 | 3 | 3 | 3 | - | - |
| Magnesium Stearate | - | - | - | - | - | - | 4.0 | 4.2 |

### <Comparative Examples 1∼2> Production of udenafil granules using typical granulating agent

In order to compare the granules according to the present invention and the fluidity thereof, granules were produced in the same manner as in Examples 1∼4 using components shown in Table 4 below, and defined as Comparative Examples 1 and 2.

**TABLE 4**

| unit: g | C.Ex.1 | C.Ex.2 |
|---|---|---|
| Udenafil | 75 | 75 |
| Anhydrous Citric Acid | 9.4 | 9.4 |
| Lactose | 225 | - |
| Microcrystalline Cellulose | - | 225 |

### <Comparative Examples 3∼6> Production of udenafil controlled-release preparation not adsorbed with organic acid

Components shown in Table 5 below, including udenafil, lactose, and microcrystalline cellulose, were mixed using a high-rate mixer, after which a solution of HPC-LF in ethanol was added in droplets to the above mixture powder, thus producing granules which were than dried using a high-rate dryer at 60°C and sieved. To the granules thus obtained, hydroxypropylmethylcellulose 4000SR which is a water-soluble release control material was added such that the amount thereof was 7.5, 10, 15, 20% per tablet, and then Cellactose 80 was added thereto and mixed together, after which 1.9 parts by weight of sodium stearyl fumarate was further added and mixed. The resulting mixture was molded into 10KP using a tablet compressor so that the amount of main component was 75 mg per tablet, thus forming tablets, and defined as Comparative Examples 3∼6.

**TABLE 5**

| unit: g | C.Ex.3 | C.Ex.4 | C.Ex.5 | C.Ex.6 |
|---|---|---|---|---|
| Udenafil | 75 | | | |
| Lactose | 37.5 | | | |
| Microcrystalline Cellulose | 28.1 | | | |
| HPC-LF | 2.8 | | | |
| HPMC 4000SR | 13.8 | 18.8 | 28.1 | 37.5 |
| Cellactose80 | 26.3 | 18.8 | 9.4 | - |
| Sodium Stearyl Fumarate | 1.9 | 1.9 | 1.9 | 1.9 |

### <Experimental Example 1> Observation of fluidity of granules

In order to evaluate whether the granules adapted for producing a controlled-release preparation were formed, the degree of granulation of the granules obtained in 1) of Examples 1 to 4 and the granules obtained in Comparative Examples 1 and 2 was evaluated with the naked eye, and the fluidity of granules after drying was determined by measuring the period of time required to pass 100 ml of the granules through a hole having a predetermined size using PTG-S3 available from Pharmatest.

As is apparent from Table 6 below, in Examples 1 to 4 in which the granules were obtained by adsorbing and granulating the udenafil-citric acid solution according to the present invention using silicon dioxide, the shape of the granules was good and the fluidity was good after drying. However, in Comparative Examples 1 and 2 in which the granules were formed using a typical excipient for granulation, it was difficult to achieve granulation attributed to stickiness, and the fluidity was also poor after drying.

**TABLE 6**

| unit | Ex. 1 | Ex.2 | Ex.3 | Ex.4 | C.Ex.1 | C.Ex.2 |
|---|---|---|---|---|---|---|
| Fluidity | 24.3 sec | 23.5 sec | 23.2 sec | 23.1 sec | 2 min or longer | 2 min or longer |
| Granulation | good | good | good | good | impossible | impossible |

### <Experimental Example 2> Evaluation of dissolution

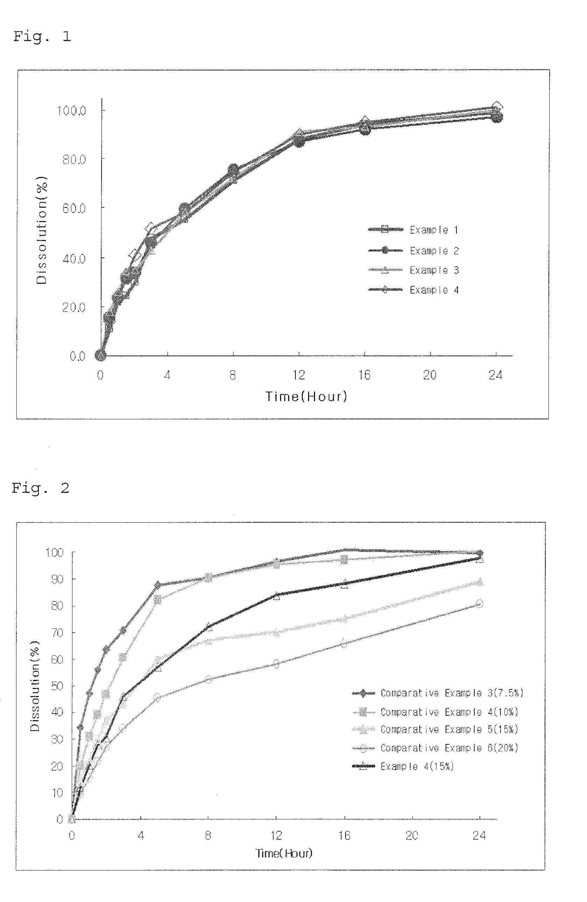

Using the method A of delayed-release dosage form by means of the second device of USP2007 Dissolution test, the dissolution of the tablets produced in Examples 1 to 4 and Comparative Examples 3 to 6 was evaluated at pH 1.2 for 2 hours and at pH 6.8 for 22 hours. The results are shown in FIGS. 1 and 2.

As shown in FIG. 1, in Examples 1 to 4 using the organic acid-adsorbed granules according to the present invention, there was no delay of dissolution depending in changes in pH regardless of the amount of solubility modulator, and initial burst could be controlled.

However, as shown in FIG. 2, when comparing the dissolution pattern in Comparative Examples 3 to 6 containing no organic acid with that of Example 4, in Comparative Examples 5 and 6 in which the amount of water-soluble polymer was increased to control initial burst, the dissolution was delayed upon change in pH to 6.8 from 1.2. In Comparative Examples 3 and 4 in which the amount of water-soluble polymer was decreased to solve the above problems, very fast release occurred at the initial stage of the dissolution, and thus these preparations were regarded as unsuitable for use as controlled-release preparations.

### <Experimental Example 3> Evaluation of dissolution at various pH values

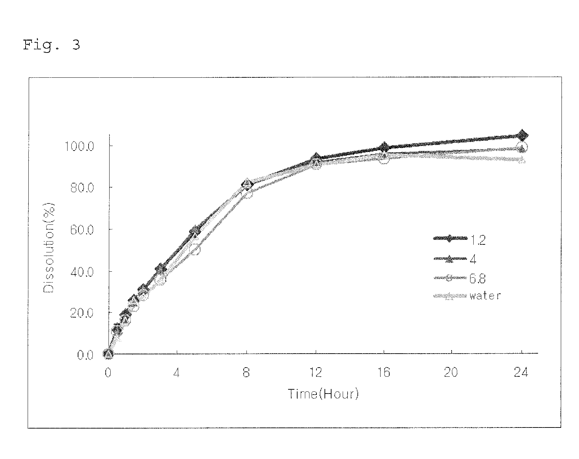

The controlled-release preparation of Example 4 was subjected to dissolution evaluation at pH 1.2, 4.0, 6.8 and in distilled water using a paddle method (50 rpm) according to the method of the Korean Pharmacopoeia. The results are shown in FIG. 3. As shown in FIG. 3, the controlled-release preparation of Example 4 could release udenafil at a predetermined rate regardless of the pH level in the wide pH range.

### <Experimental Example 4> Measurement of 80% dissolution time point of drug

In order to evaluate the dissolution of the composition according to the present invention depending on the kind of polymer, the weight ratio and the viscosity, the dissolution test was carried out in the same manner as in Test Example 2, and the time point at which 80% of the total drug was dissolved was measured. As is apparent from Table 7 below, the 80% release time could be freely controlled in the range of 3∼24 hours in various doses depending on the viscosity of polymer and the weight ratio.

**TABLE 7**

| Ex. No. | Weight of Drug | Water-soluble polymer, Weight ratio | 80% Dissolution Time Point (hour) |
|---|---|---|---|
| 4 | 75 | HPMC4000, 15% | 8∼12 |
| 5 | 75 | HPMC4000, 7.5% | 4 |
| 6 | 75 | PEO WSR 303, 40% | 20∼24 |
| 7 | 75 | PEO WSR 303, 30% | 14∼16 |
| 8 | 75 | PEO WSR 303, 15% | 12 |
| 9 | 75 | PEO WSR 303, 10% | 6 |
| 10 | 75 | PEO WSR 301, 10% | 3 |
| 11 | 100 | PEO WSR 301, 10% | 6 |
| 12 | 100 | PEO WSR 301, 10%, Sodium Starch Glycolate 10% | 3 |

## Claims

1. A controlled-release composition for producing a sustained-release preparation containing udenafil, comprising:
udenafil and its pharmaceutically acceptable salt;
a solubility modulator selected from among organic acids including citric acid, malic acid, adipic acid, maleic acid, ascorbic acid, succinic acid, and tartaric acid;
an adsorbent selected from among silicon dioxide, calcium silicate, talc, and aluminum magnesium metasilicate; and
a hydrophilic polymer selected from among hydroxyethylcellulose, hydroxypropylcellulose, hydroxypropylmethylcellulose, polyethyleneoxide, xanthan gum, guar gum, locust bean gum, and sodium alginate.

2. The controlled-release composition according to claim 1, wherein the solubility modulator is citric acid.

3. The controlled-release composition according to claim 1, wherein the adsorbent is silicon dioxide.

4. The controlled-release composition according to claim 1, wherein the hydrophilic polymer is selected from among hydroxypropylmethylcellulose and polyethyleneoxide.

5. The controlled-release composition according to claim 1, further comprising a pore forming agent.

6. The controlled-release composition according to claim 1, further comprising a swelling agent.

7. The controlled-release composition according to claim 1, further comprising a diluent.

8. The controlled-release composition according to claim 1, further comprising a lubricant.

9. A method of preparing a controlled-release composition for producing a sustained-release preparation containing udenafil, comprising:
adsorbing a mixture solution comprising udenafil and its pharmaceutically acceptable salt, and a solubility modulator selected from among organic acids including citric acid, malic acid, adipic acid, maleic acid, ascorbic acid, succinic acid, and tartaric acid onto a surface of an adsorbent selected from among silicon dioxide, calcium silicate, talc, and aluminum magnesium metasilicate, thus preparing adsorbed granules; and
mixing the adsorbed granules with a hydrophilic polymer selected from among hydroxyethylcellulose, hydroxypropylcellulose, hydroxypropylmethylcellulose, polyethyleneoxide, xanthan gum, guar gum, locust bean gum, and sodium alginate.
